(19) Europäisches Patentamt
European Patent Office
Office européen des brevets

(11) Veröffentlichungsnummer: **0 002 222**
**B1**

(12) **EUROPÄISCHE PATENTSCHRIFT**

(21) Anmeldenummer: **78101404.8**

(22) Anmeldetag: **18.11.78**

(51) Int. Cl[3]: **C 07 D 211/74,**
A 01 N 43/40

# (54) Aralkylpiperidinone und deren Anwendung als Fungizide

UNGÜLTIG

(30) Priorität: **30.11.77 DE 2753278**

(43) Veröffentlichungstag der Anmeldung:
**13.06.79 Patentblatt 79/12**

(45) Bekanntmachung des Hinweises auf die Patenterteilung:
**28.05.80 Patentblatt 80/11**

(84) Benannte Vertragsstaaten:
**BE CH DE FR GB LU NL SE**

(56) Entgegenhaltungen:
**Keine**

(73) Patentinhaber: **BASF Aktiengesellschaft**
**Carl-Bosch-Strasse 38**
**D - 6700 Ludwigshafen (DE)**

(72) Erfinder: **Goetz, Norbert, Dr.**
**Schoefferstrasse 25**
**D - 6520 Worms (DE)**
**Himmele, Walter, Dr.**
**Eichenweg 14**
**D - 6909 Walldorf (DE)**
**Zeeh, Bernd, Dr.**
**Thorwaldstrasse 5**
**D - 6700 Ludwigshafen (DE)**
**Pommer, Ernst-Heinrich, Dr.**
**BerlinerPlatz 7**
**D - 6703 (DE)**

(74) Vertreter:

**EP 0 002 222 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Aralkylpiperidinone und deren Anwendung als Fungizide

Die vorliegende Erfindung betrifft wertvolle neue Aralkyl-3,5-dimethyl-piperidin-4-one und ihre Salze, Fungizide, die diese Verbindungen enthalten, und Verfahren zur Bekämpfung von Pilzen mit diesen Verbindungen.

Aus den deutschen Patentschriften 1 164 152, 1 173 722, 1 198 125 und der DE-OS 2 461 513 ist es bekannt, N-alkyl-substituierte Morpholine und ihre Salze als Fungizide zu verwenden.

Es wurde gefunden, daß 3,5-Dimethyl-piperidin-4-on-Derivate der allgemeinen Formel

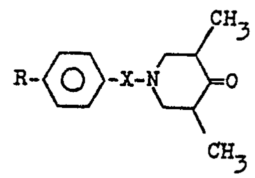

in der X einen geraden oder verzweigten Alkylenrest mit 3 bis 10 Kohlenstoffatomen, R eine Alkyl- oder Alkoxygruppe mit 1 bis 8 Kohlenstoffatomen, vorzugsweise eine Isopropyl- oder Tertiärbutylgruppe, bedeutet, und ihre Salze eine gute fungizide Wirkung besitzen, die der Wirkung der bekannten Morpholinderivate überlegen ist.

Als Salze kommen solche mit anorganischen oder organischen Säuren in Frage, wie beispielsweise mit Halogenwasserstoffsäuren (Salzsäure, Bromwasserstoffsäure), Schwefelsäure, Saltpetersäure, Phosphorsäure, Ameisensäure, Essigsäure, Propionsäure, Buttersäure, Acrylsäure, Oxalsäure, Adipinsäure, Milchsäure, Weinsäure, Citronensäure, Trichloressigsäure, Stearinsäure, Ölsäure, p-Toluolsulfonsäure, Dodecylbenzolsulfonsäure, Dodecansulfonsäure oder Perfluoroctansäure.

X bedeutet beispielsweise

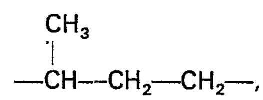

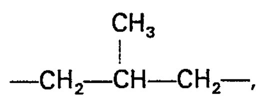

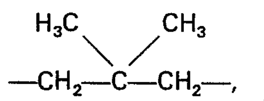

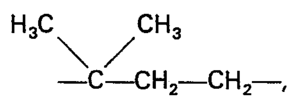

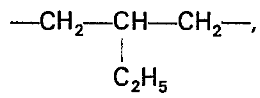

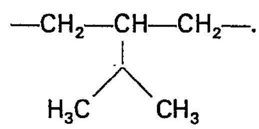

R bedeutet beispielsweise Methyl, Äthyl, Isopropyl, Butyl, sec.-Butyl, tert.-Butyl, Methoxy, Äthoxyl, Isopropoxyl.

Die Herstellung der erfindungsgemäßen N-substituierten 3,5-Dimethylpiperidin-4-one kann durch Umsetzung der entsprechenden primären Amine mit 2,4-Dimethylpenta-1,4-dien-4-on vorgenommen werden:

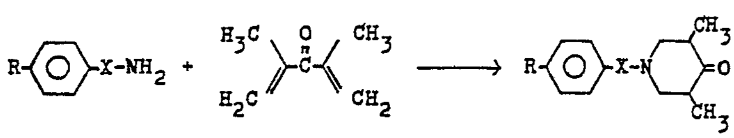

wobei R und X die oben angegebene Bedeutung haben. Die benötigten primären Amine lassen sich durch reduktive Aminierung aus den entsprechenden Aldehyden bzw. Ketonen herstellen (Houben-Weyl "Methoden der organischen Chemie", Band 11/1, S. 602 ff). Das 2-Methyl-3-(p-tert.-butylphenyl)-propylamin kann beispielsweise nach folgender Vorschrift hergestellt werden.

800 Teile (Gewichtsteile) 2-Methyl-3-(p-tert.-butylphenyl)-propanal werden in 800 Teilen Methanol gelöst und zusammen mit 150 Teilen Raney-Nickel in einen 3 l Hydrierautoklaven eingefüllt. Es werden 540 Teile Ammoniak aufgepreßt, dann wird die Reaktionsmischung auf 90°C erhitzt. Bei 90°C wird solange Wasserstoff aufgepreßt, bis ein Gesamtdruck von 100 bar erreicht ist. Mit diesem konstanten Druck wird 30 Stunden lang hydriert.

Das Reaktionsprodukt wird anschließend filtriert und das Filtrat destillativ aufgearbeitet. Hierbei erhält man 750 Teile (≙ 93 % d.Th.) 2-Methyl-3-(p-tert.-butylphenyl)-propylamin, $Kp_{0,01}$ = 97°C.

Die Synthese des 2,4-Dimethylpenta-1,4-dien-3-ons kann aus Diisopropylketon durch Bromierung und Abspaltung von Bromwasserstoff (O. Sorokin, I zv. Akad. Nauk SSSR 1961, 460—466) oder nach Houben-Weyl "Methoden der organischen Chemie" Band 7/2 a, S. 743 bzw. S. F. Reed, J. Org. Chem. 27, S. 4 116 (1962) erfolgen.

Von den erfindungsgemäßen Verbindungen sind beispielsweise die folgenden in einer Tabelle zusammengestellt:

| Nr. | Verbindung (Y = 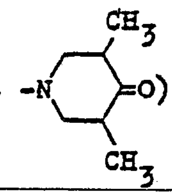) | Kp (°C)/Torr | Fp (°C) |
|---|---|---|---|
| 1 | 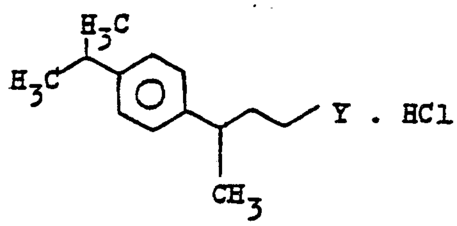 | | 188 |
| 2 | 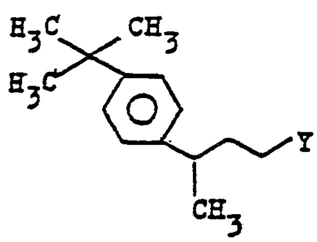 | 152/0,5 | |
| 3 | 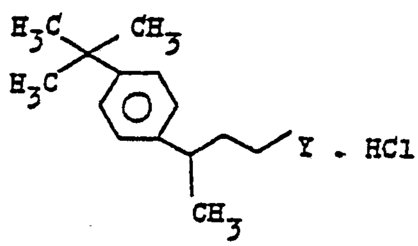 | | 200 |
| 4 | 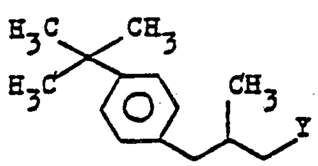 | 187/5,0 | |
| 5 | 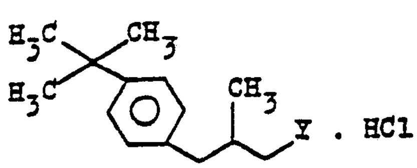 | | 210 |

| Nr. | Verbindung | Kp (°C)/Torr | Fp (°C) |
|---|---|---|---|
| | 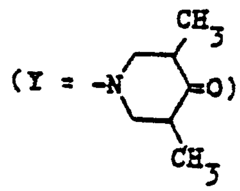 | | |
| 6 | 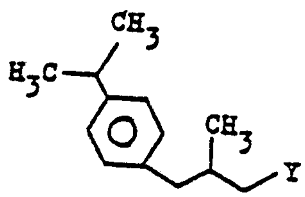 | 205/5 | |
| 7 | 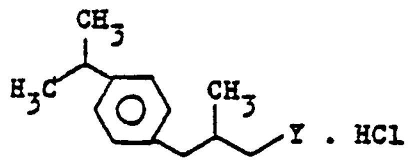 | | 184 |
| 8 | 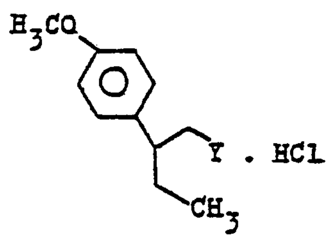 | | 200 |
| 9 | 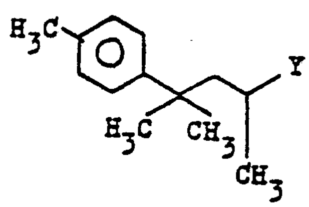 | 143/0,01 | |
| 10 | 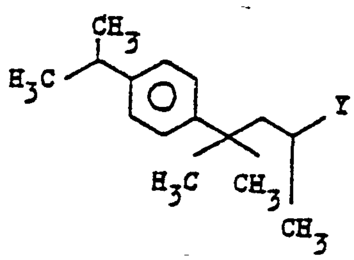 | 155/0,01 | |
| 11 | 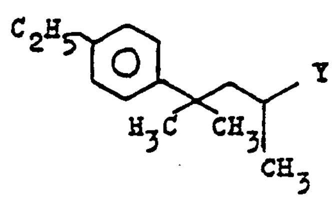 | 140/0,02 | |
| 12 | 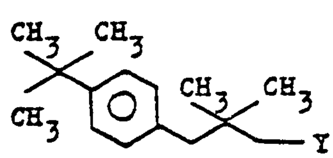 | 162/0,01 | |

| Nr. | Verbindung (Y = 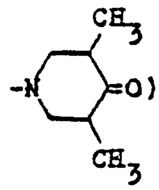) | Kp (°C)/Torr | Fp (°C) |
|---|---|---|---|
| 13 | 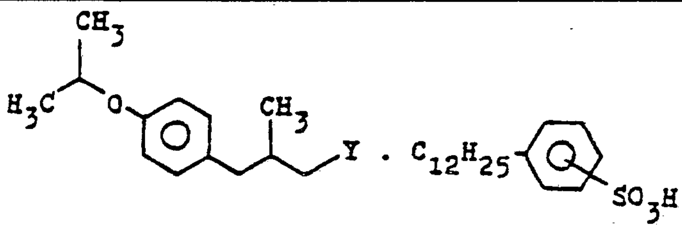 | | 108 |
| 14 | 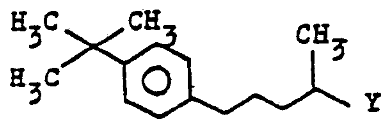 | 158/0,01 | |
| 15 | 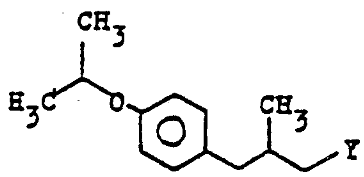 | 154/0,05 | |
| 16 | 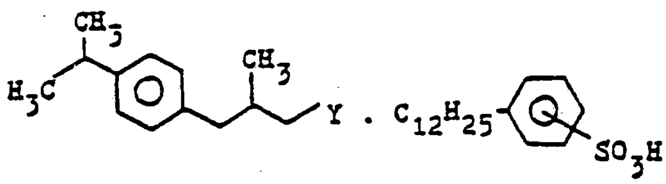 | | 110 |
| 17 | 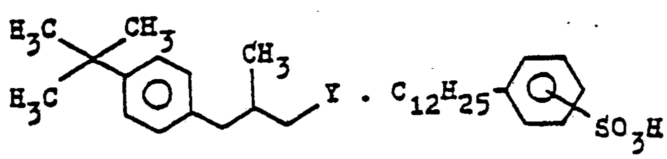 | | 102 |
| 18 | 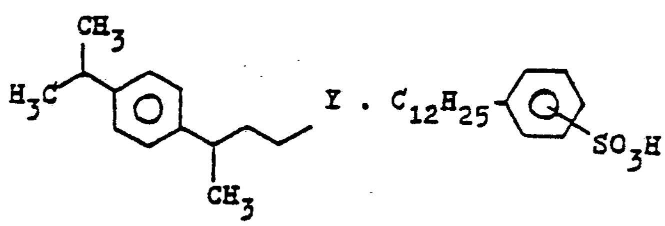 | | 121 |
| 19 | 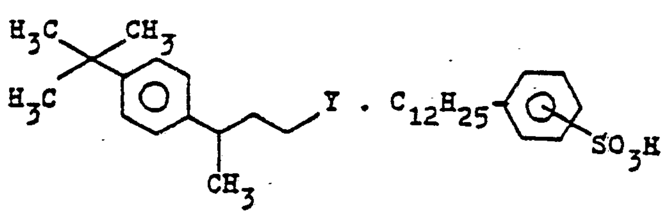 | | 112 |
| 20 | 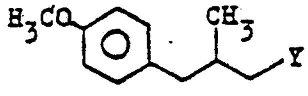 | 160/0,01 | |

Herstellungsbeispiele

BEISPIEL 1

25 Teile (Gewichtsteile) 3-(p-tert.-Butylphenyl)-2,2-dimethylpropylamin werden in 100 Teilen Methanol gelöst. Bei Raumtemperatur wird eine Lösung von 12,6 Teilen 2,4-Dimethyl-penta-1,4-dien-3-on in 30 Teilen Methanol zugegeben und anschließend 5 Stunden lang bei Rückflußtemperatur (65°C) erhitzt. Das Reaktionsprodukt wird destillativ aufgearbeitet, wobei 32 Teile (≙ 85 % d.Th.) N-[3-(p - tert. - Butylphenyl) - 2,2 - dimethylpropyl] - 3,5 - dimethyl - piperidin - 4 - on erhalten werden, $Kp_{0,01}$ = 162°C. (Verbindung Nr. 12).

BEISPIEL 2

6,3 Teile N-[2-(p-tert.-Butylphenyl)-propyl]-3,5-dimethyl-piperidin-4-on werden in 50 Teilen Methanol gelöst und bei Raumtemperatur mit 32,5 Raumteilen einer 0,5 molaren methanolischen Dodecylbenzolsulfonsäure-Lösung auf einen pH-Wert von 5 eingestellt. Anschließend wird das Lösungsmittel vollständig abdestilliert und man erhält 10,5 Teile (≙ 82 % d.Th.) des dodecylbenzolsulfonsauren Salzes vom N-[2-(p-tert.-Butylphenyl)-propyl]-3,5-dimethyl-piperidin-4-on, Fp. 112°C. (Verbindung Nr. 19).

Die erfindungsgemäßen Wirkstoffe und die entsprechenden Fungizide sind insbesondere geeignet zur Bekämpfung von Pflanzenkrankheiten, z.B. Erysiphe graminis (echter Mehltau) an Getreide, Erysiphe cichoriacearum (echter Mehltau) an Kürbisgewächsen, Podosphaera leucotricha an Äpfeln, Uncinula necator an Reben, Erysiphe polygoni an Bohnen. Sphaerotheca pannosa an Rosen, Microsphaera querci an Eichen. Mycosphaerella musicola an Bananen, Puccinia-Arten (Rostpilze) an Getreide, Uromyces appendiculatus und U. phaseoli an Bohnen, Hemileia vastatrix an Kaffee und Rhizoctonia solani. Sie sind systematisch wirksam; sie werden sowohl über die Wurzeln als auch über die Blätter aufgenommen und im Pflanzengewebe transportiert.

Bei der Anwendung der neuen Wirkstoffe zur Behandlung von Pflanzen gegen Pilzinfektionen liegen die Aufwandmengen zwischen 0,025 und 5 kg Wirkstoff/ha Fläche. Zum Oberflächenschutz von Bäumen oder Früchten können die Wirkstoffe auch in Verbindung mit Kunststoffdispersionen 0,25 %ig bis 5 %ig, bezogen auf das Gewicht der Dispersion, verwendet werden. Die Fungizide enthalten im allgemeinen zwischen 0,1 und 95 Gewichtsprozent Wirkstoff, vorzugsweise zwischen 0,5 und 90 %.

Die Wirkstoffe können mit anderen bekannten Fungiziden gemischt werden. In vielen Fällen erhält man dabei eine Vergrößerung des fungiziden Wirkungsspektrums; bei einer Anzahl von Fungizidmischungen in den Gewichtsverhältnissen 1:10 bis 10:1 treten auch synergistische Effekte auf, d.h. die fungizide Wirksamkeit des Kombinationsproduktes ist größer als die der addierten Wirksamkeiten der Einzelkomponenten. Fungizide, die mit den erfindungsgemäßen Verbindungen kombiniert werden können, sind beispielsweise:

Dithiocarbamate und deren Derivate, wie
Zinkdimethyldithiocarbamat,
Manganäthylenbisdithiocarbamat,
Mangan-Zink-äthylendiamin-bis-dithiocarbamat,
Zinkäthylenbisdithiocarbamat,
Tetramethylthiuramdisulfid,
Ammoniak-Komplex von Zin-(N,N-äthylen-bis-dithiocarbamat)
und
N,N'-Polyäthylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und N,N'-Polypropylen-bis-(thiocarbamoyl)-disulfid;
heterocyclische Verbindungen, wie
N-Trichlormethylthio-tetrahydrophthalimid,
N-Trichlormethylthio-phthalimid,
N-(1,1,2,2-Tetrachloräthylthio)-tetrahydroththalimid,
1-(Butylcarbamoyl)-2-benzimidazol-carbaminsäuremethylester,
2-Methyloxycarbonylamino-benzimidazol,
2,3-Dihydro-5-carboxanilido-6-methyl-1,4-oxathiin-4,4-dioxid,
2,3-Dihydro-5-carboxanilido-6-methyl-1,4-oxathiin,
5-Butyl-2-dimethylamino-4-hydroxy-6-methyl-pyrimidin,
1,2-Bis-(3-äthoxycarbonyl-2-thioureido)-benzol,
1,2-Bis-(3-methoxycarbonyl-2-thioureido)-benzol
und verschiedene andere Fungizide, wie
Dodecylguanidinacetat,
N-Dichlorfluormethylthio-N',N'-dimethyl-N-phenyl-schwefelsäurediamid,

2,5-Dimethyl-furan-3-carbonsäureanilid,
2,5-Dimethyl-furan-3-carbonsäure-cyclohexylamid,
2-Jodbenzoesäureanilid,
2-Brombenzoesäureanilid,
3-Nitro-isophthalsäure-diisopropylester,
1-(1,2,4-Triazolyl-1')-[1-(1-(4'-chlorphenoxy)]-3,3-dimethylbutan-2-on,
1-(1-Imidazolyl)-2-allyloxy-2-(2,4-dichlorphenyl)-äthan,
Piperazin-1,4-diyl-bis-1-(2,2,2-Trichloräthyl)-formamid,
2,4,5,6-Tetrachlor-isophthalonitril,
1,2-Dimethyl-3,5-diphenyl-pyrazoliniummethylsulfat,
ß-(4-Chlorphenoxy)-$\alpha$-(1,1-dimethyl)-1H,1,2,4-triazol-1-äthanol.

Die Anwendung erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate, zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, z.B. Dimethylformamid, Dimethylsulfoxid, N-Methylpyrrolidon, Wasser usw. in Betracht.

Waßrige Anwendungsformen können uas Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberflächenaktiven Stoffen sind zu nennen:

Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylen-octylphenoläther, äthoxyliertes Isooctylphenol-, Octylphenol-, Nonylphenol, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetat, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatemeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat Harnstoffe und pflanzliche Produkte, wie Getreidemehl, Baumrinden-, Holz und Nußschalenmehl, Cellulosepulver und andere feste Trägerstoffe.

Zu den Mischungen oder Einzelwirkstoffen können Öle verschiedenen Typs, Herbizide, Fungizide, Nematozide, Insektizide, Bakterizide, Spurenelemente, Düngemittel, Antischaummittel (z.B. Silikone), Wachstumsregulatoren, Antidotmittel oder andere wirksame Verbindungen, zugesetzt werden.

Für die folgenden Versuche wurden folgende bekannte Vergleichssubstanzen verwendet.

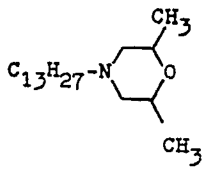

Wirkstoff Nr. 21
(DE-PS 1 164 152)

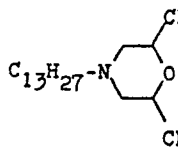 · $CH_3COOH$

Wirkstoff Nr. 22
(DE-PS 1 173 722)

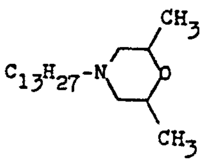 · 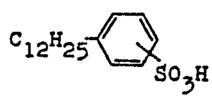

Wirkstoff Nr. 23
(DE-OS 2 461 513)

## BEISPIEL 3

Blätter von in Töpfen gewachsenen Weizenkeimlingen der Sorte "Jubilar" werden mit wäßrigen Emulsionen aus 80 % (Gewichtsprozent) Wirkstoff und 20 % Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22°C und 75 bis 80 % relativer Luftfeuchtigkeit aufgestellt. Nach 10 Tagen wird das Ausmaß der Mehltaupilzentwicklung ermittelt.

| Wirkstoff | Befall der Blätter nach Sprintzungen mit x %iger Wirkstoffbrühe x = 0,025 | 0,05 |
|---|---|---|
| 3 | 0 | 0 |
| 4 | 2 | 0 |
| 5 | 2 | 0 |
| 6 | 0 | 0 |
| 7 | 0 | 0 |
| 8 | 0 | 0 |
| 14 | 0 | 0 |
| 15 | 0 | 0 |
| 16 | 1 | 0 |
| 17 | 0 | 0 |
| 19 | 1 | 0 |
| 21 } bekannt | 2 | 1 |
| 22 } bekannt | 2 | 1 |
| Kontrolle (unbehandelt) | 5 | |

0 kein Befall, abgestuft bis 5 Totalbefall

BEISPEIL 4

In einem weiteren Versuch werden, entsprechend wie in Beispiel 3 beschrieben, Blätter von in Töpfen gewachsenen Gerstenkeimlingen der Sorte "Firlbecks Union" behandelt und mit Sporen (Konidien) des Gerstenmehltaus (Erysiphe graminis var. hordei) bestäubt.

| Wirkstoff | Befall der Blätter nach Spritzungen mit x %iger Wirkstoffbrühe x = 0,025 | 0,05 |
|---|---|---|
| 2 | 0 | 0 |
| 3 | 0 | 0 |
| 4 | 0 | 0 |
| 5 | 0 | 0 |
| 7 | 0 | 0 |
| 12 | 0 | 0 |
| 14 | 0 | 0 |
| 15 | 0 | 0 |
| 16 | 0 | 0 |
| 17 | 0 | 0 |
| 18 | 0 | 0 |
| 19 | 0 | 0 |
| 20 | 0 | 0 |
| 22 bekannt | 1 | 1 |
| Kontrolle (unbehandelt) | 5 | |

0 kein Befall, abgestuft bis 5 Totalbefall

BEISPIEL 5

Blätter von in Töpfen gewachsenen Weizenpflanzen werden mit Sporen des Weizenbraunrostes (Puccinia recondita) künstlich infiziert und 48 Stunden lang bei 20 bis 25°C in einer wasserdampfgesättigten Kammer aufgestellt. Danach werden die Pflanzen mit wäßrigen Spritzbrühen, die in dem Wasser gelöst oder emulgiert eine Mischung aus 80 % des zu prüfenden Wirkstoffes und 20 % Natriumligninsulfonat enthalten, besprüht und im Gewächshaus bei Temperaturen zwischen 20 und 22°C und bei 75 bis 80 % relativer Luftfeuchtigkeit aufgestellt. Nach 10 Tagen wird das Ausmaß der Rostpilzentwicklung beurteilt.

| Wirkstoff | Befall der Blätter nach Spritzungen mit x %iger Wirkstoffbrühe x = 0,05 | 0,1 |
|---|---|---|
| 3 | 2 | 1 |
| 4 | 0 | 0 |
| 5 | 0 | 0 |
| 6 | 0 | 0 |
| 7 | 0 | 0 |
| 16 | 0 | 0 |
| 17 | 0 | 0 |
| 21 } bekannt | 3 | 2 |
| 22 } bekannt | 4 | 3 |
| 23 } bekannt | 3 | 3 |
| Kontrolle (unbehandelt) | 5 | |

0 kein Befall, abgestuft bis 5 Totalbefall

BEISPIEL 6

Man vermischt 90 Gewichtsteile der Verbindung 1 mit 10 Gewichtsteilen N-Methyl-$\alpha$-pyrrolidon und erhält eine Lösung, die zur Anwendung in Form kleinster Tropfen geeignet ist.

BEISPIEL 7

20 Gewichsteile der Verbindung 2 werden in einer Mischung gelöst, die aus 80 Gewichsteilen Xylol, 10 Gewichtsteilen des Anlagerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsprodktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

BEISPIEL 8

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

BEISPIEL 9

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

BEISPIEL 10

20 Gewichtsteile des Wirkstoffs 2 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

BEISPIEL 11

3 Gewichtsteile der Verbindung 3 werden mit 97 Gewichtsteilen feinteiligem Kaolin innig vermischt. Man erhält auf diese Weise ein Stäubemittel, das 3 Gewichtsprozent des Wirkstoffs enthält.

BEISPIEL 12

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufarbeitung des Wirkstoffs mit guter Haftfähigkeit.

BEISPIEL 13

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

BEISPIEL 14

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

**Patentansprüche**

1. N-substituierte 3,5-Dimethyl-piperidin-4-on-Derivate der allgemeinen Formel

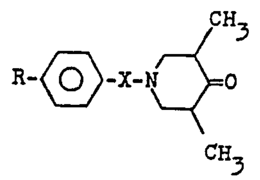

in der X einen geraden oder verzweigten Alkylenrest mit 3 bis 10 Kohlenstoffatomen, R eine Alkyl- oder Alkoxy-gruppe mit 1 bis 8 Kohlenstoffatomen bedeutet, und die Salze dieser Verbindungen.

2. Fungizide Mittel, enthaltend eine Verbindung gemäß Anspruch 1.

3. N-substituiertes 3,5-Dimethyl-piperidin-4-on-Derivat, ausgewählt aus der Gruppe bestehend aus

N - [3 - (p - Isopropyl - phenyl) - 2 - methyl - propyl] - 3,5 - dimethyl - piperidin - 4 - on,

N - [3 - (p - Isopropyl - phenyl) - 2 - methyl - propyl] - 3,5 - dimethyl - piperidin - 4 - on. Hydrochlorid,

N - [2 - (p - Methoxy - phenyl) - butyl] - 3,5 - dimethyl - piperidin - 4 - on,

N - [4 - (p - tert. - Butyl - phenyl) - 1 - methyl - butyl] - 3,5 - dimethyl - piperidin - 4 - on,

N - [3 - (p - Isopropoxy - phenyl) - 2 - methyl - propyl] - 3,5 - dimethyl - piperidin - 4 - on,

N - [3 - (p - tert. - Butyl - phenyl) - 2 - methyl - propyl] - 3,5 - dimethyl - piperidin - 4 - on. Dodecylbenzosulfonat.

**Claims**

1. N-substituted 3,5-dimethyl-piperidin-4-one derivatives of the general formula

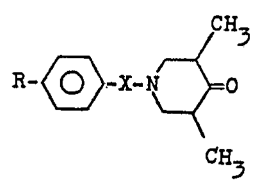

where X is a linear or branched alkylene radical of 3 to 10 carbon atoms, R is an alkyl or alkoxy group of 1 to 8 carbon atoms, and the salts of these compounds.

2. Fungicidal agents containing a compound as claimed in claim 1.

3. N-substituted 3,5-dimethyl-piperidin-4-one derivative selected from the group consisting of

N - [3 - (p - isopropyl - phenyl) - 2 - methyl - propyl] - 3,5 - dimethyl - piperidin - 4 - one,

N - [3 - (p - isopropyl - phenyl) - 2 - methyl - propyl] - 3,5 - dimethyl - piperidin - 4 - one. Hydrochloride,

N - [2 - methoxy - phenyl) - butyl] - 3,5 - dimethyl - piperidin - 4 - one,

N - [4 - (p - tert. - butyl - phenyl) - 1 - methyl - butyl] - 3,5 - dimethyl - piperidin - 4 - one,

N - [3 - p - isopropoxy - phenyl) - 2 - methyl - propyl] - 3,5 - dimethyl - piperidin - 4 - one,

N - [3 - (p - tert. - butyl - phenyl) - 2 - methyl - propyl] - 3,5 - dimethyl - piperidin - 4 - one. Dodecylbenzene sulfonate.

**Revendications**

1. Dérivés N-substitués de la 3,5-diméthyl-pipéridine-4-one de la formule générale

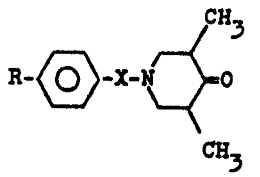

dans laquelle le symbole X représente un pont alcoylène en $C_3$ à $C_{10}$ et le symbole R désigne un radical alcoyle ou alcoxy en $C_1$ à $C_8$, et les sels de ces dérivés.

2. Composition fongicide, contenant un composé suivent la revendication 1.

3. Dérivé N-substitué de la 3,5-diméthyl-pipéridine-4-one choisi parmi:

la N - [3 - p - isopropyl - phényl) - 2 - méthyl - propyl] - 3,5 - diméthyl - pipéridine - 4 - one,

le chlorhydrate de la N - [3 - (p - isopropyl - phényl) - méthyl - propyl] - 3,5 - diméthyl-pipéridine - 4 - one,

la N - [2 - (p - méthoxy - phényl) - butyl] - 3,5 - diméthyl - pipéridine - 4 - one,

la N - [4 - (p - tertiobutyl - phényl) - 1 - méthyl - butyl] - 3,5 - diméthyl - pipéridine - 4 - one,

la N - [3 - (p - isopropoxy - phényl) - 2 - méthyl - propyl] - 3,5 - diméthyl - pipéridine - 4 - one et

le dodécyl - benzène - sulfonate de la N - [3 - (p - tertiobutylphényl) - 2 - méthyl - propyl] - 3,5-diméthyl - pipéridine - 4 - one.